# EUROPEAN PATENT SPECIFICATION

(11) **EP 0 540 357 B1**
(45) Date of publication and mention of the grant of the patent: **24.07.1996**
(21) Application number: 92309969.1
(22) Date of filing: 30.10.1992
(51) Int. Cl.: C08H 1/00, A61K 7/06

(54) **Protein-silicone copolymers and compositions containing them**
Copolymere aus Protein und Siloxan und diese enthaltende Zusammensetzungen
Copolymères protéine-silicones et compositions les contenant

(30) Priority: 01.11.1991 GB 9123251
(43) Date of publication of application: 05.05.1993
(73) Proprietor: CRODA INTERNATIONAL plc, Goole North Humberside DN14 9AA (GB)
(72) Inventor: Jones, Roger T., Chuddington, Cheshire CW8 2EE (GB); Humphries, Mark A., Warrington, Cheshire WA5 5SF (GB)
(74) Representative: Allden, Thomas Stanley

(56) References cited:
- GB-A- 2 159 408
- US-A- 3 661 584
- US-A- 4 713 116
- US-A- 5 100 956
- PATENT ABSTRACTS OF JAPAN vol. 15, no. 508 (C-897)(5036) 24 December 1991 & JP-A-32 23 207
- PATENT ABSTRACTS OF JAPAN vol. 13, no. 516 (C-656)(3864) 17 November 1989 & JP-A-12 11 517

## Description

This invention relates to certain new protein-silicone copolymers and to their use in cosmetics and toiletries.

The use of proteins, their hydrolysates and chemically modified derivatives in cosmetic formulations to provide performance and conditioning benefits to skin and hair is well known. These benefits derive mainly from the film-forming and moisture-retentive properties exhibited by such "cosmetic proteins" together with their substantivity to hair and skin, that is their ability to adsorb to hair and skin and to resist elution with water.

Proteins from a variety of animal and vegetable sources can be used and the relative film-forming and moisture-retentive propeties can be controlled by the selection of a particular protein and its average molecular weight. It is also possible to chemically modify proteins to modify their behaviour and functionality; such chemical modification includes, for example, esterification of carboxyl groups, acylation of amino groups and quaternisation of amino groups.

Benefits claimed for proteins and their derivatives in the conditioning of hair include improved combability, gloss, moisture control, body and reduced 'fly-away'. For skin, benefits include moisturisation, skin-firming/smoothing, improved feel and anti-irritation effect.

Another category of conditioning agents which have established a significant role in cosmetics products are silicones. "Silicone" is a general term for a range of chemicals with different behaviour and effects but characterised by the "silicon-oxygen-silicon" chain. Polydimethylsiloxanes (dimethicones) exhibit low solubility in water, low surface tension and antifoam behaviour and have been found to confer gloss and lubricity to hair and a soft feel to skin. To overcome the limitations of water insolubility, silicone glycols (dimethicone polyols) have been developed which also exhibit foaming behaviour and confer lubricity to keratin substrates.

In order to provide substantivity to hair, amino functional silicones (e.g. amodimethicones) have been developed which are also capable of polymerising on the hair, but are water-insoluble.

From the foregoing it will be understood that "proteins" and "silicones" represent two distinct categories of valuable conditioning agents for skin and hair with different physical properties and functional benefits.

The possibility of developing modified proteins which would have some properties characteristic of silicones (e.g. increased lubricity) or alternatively silicones with some of the benefits of proteins (greater water solubility and substantivity), offers potential for the formulation of cosmetic products with novel properties.

Products which are mixtures of silicones and proteins have been proposed and marketed, but it will be obvious to those skilled in the art of cosmetic formulations that mixtures will not provide the desired improvement in solubility characteristics or substantivity and lubricity for rinse-off systems.

European Patent 0295 983 describes products formed by the condensation of a silane, silanol or siloxane with the carboxylic acid group of amino acids. The products, which contain only one silicon atom per molecule, are claimed to have useful therapeutic and cosmetic effects.

JP-A-3223207 describes certain peptide-modified silicone derivatives, which are not cross-linked, and their use as cosmetic bases.

We have now developed a range of cross-linked protein-silicone copolymers in which the silicone component is covalently bonded to amino groups of the protein and which provide the benefits, that is the desirable physical characteristics, of both proteins and of silicones, for the conditioning of skin and/or hair which are not achievable with simple mixtures or with non-crosslinked products.

According to the present invention, there is provided a protein-silicone copolymer wherein the silicone component is covalently bonded to amino groups of the protein and at least some of the silicone component forms cross-links between different protein chains, the protein component forming from 5% to 98% by weight of the copolymer.

The present invention also comprises a cosmetic formulation, especially but not exclusively for the treatment of the skin and/or the hair, which comprises a protein-silicone copolymer according to the invention.

The copolymers according to the invention can be prepared by covalently attaching organofunctional silane/silicone compounds to the protein amino groups to form larger polymer molecules including protein cross-linking. In addition, further polymerisation may occur through condensation of silanol groups and such further polymerisation increases the amount of cross-linking.

The protein may be derived from either animal or vegetable sources or by fermentation. It may be in the form of a chemically modified protein (for example quaternised) provided that some free amino groups are still present on the protein molecules. Examples of proteins which are currently used in cosmetic formulations and which can be used as the protein component of the copolymer, include collagen, elastin, keratin, casein, wheat protein, soya protein, and silk. In this specification, the term "protein" is used to include both native and hydrolysed proteins and it thus comprises both proteins properly so-called and polypeptides, peptides and peptones since the latter can all be categorised as hydrolysed proteins.

The average molecular weight of the protein component may be from 500 to 500,000 Daltons, that is preferably within the range of from 500 to 50,000 Daltons and is more preferably within the range of from 1000 to 20,000 Daltons, expressed as weight average molecular weight derived from size exclusion chromatography.

It is necessary for the protein component to be capable of solution in water or other suitable solvent or co-solvent (such as an alcohol, propylene glycol, or polyethylene glycol) to enable reaction to occur.

The organofunctional silicone used for reaction with the protein component to form the copolymer must contain a functional group capable of reacting with the chain terminal and/or side chain amino groups of the protein. Suitable reactive groups include, for example, acyl halide, sulphonyl halide, anhydride, aldehyde and epoxide groups. The silicone component may be any compound which contains a siloxane group (Si-O-Si) or any silane capable of forming a siloxane in situ by condensation of silanol (Si-OH) groups (see Reaction 1 below) or any alkoxysilane or halosilane which hydrolyses to form a corresponding silanol (see Reaction 2 below) and then condenses to form a siloxane group (Reaction 1).

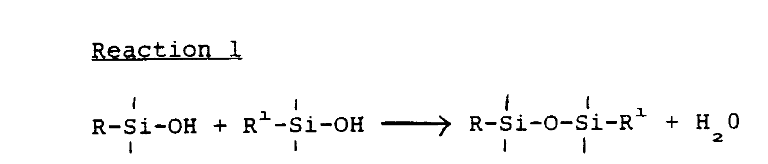

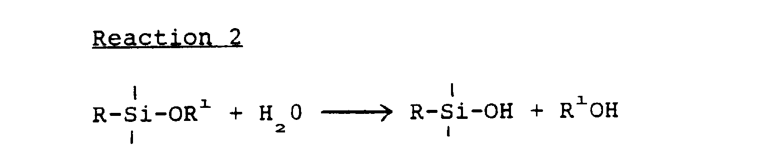

The silicone reactant must be capable of forming cross-links with the protein component. Crosslinking may be obtained either through the use of polyfunctional silicone reactants or of monofunctional silicone reactants containing silanol groups (or alkoxysilane or halosilane groups convertible to silanol groups by hydrolysis) capable of forming siloxane cross-links by condensation between different chains. Such monofunctional silicone reactants should therefore have at least one and up to three hydroxy groups attached to at least one silicon atom in their structure. Generally, but not necessarily, the silanol group(s) will be in a part of the reactant molecule remote from the amino-reactive organofunctional group.

Examples of suitable organofunctional silicone/silane reactants include compounds I-IV below: 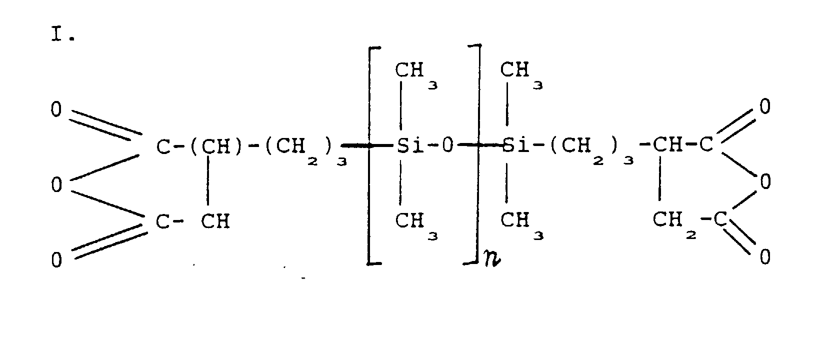 where n = 1-50, preferably 30-40. 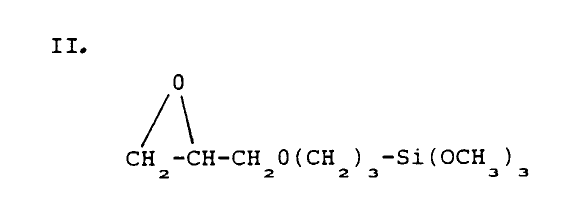 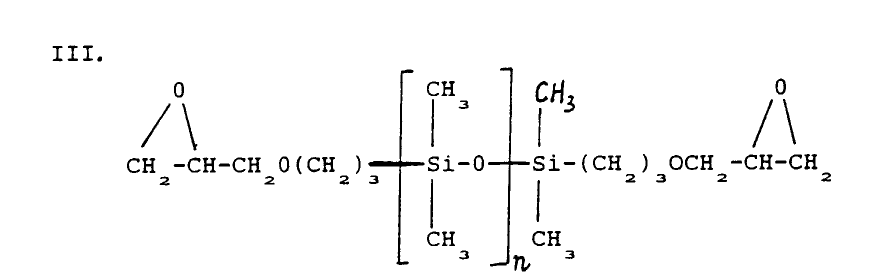 where n = 1-50, preferably 12-25.

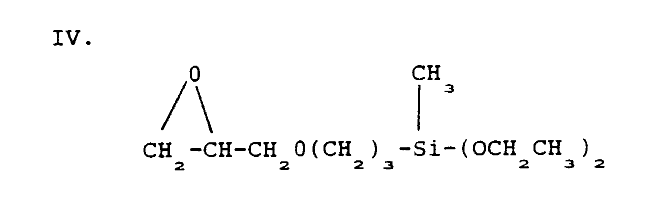

Reactants I and III are examples of difunctional silicones capable of directly producing cross-links with protein amino groups. Reactants II and IV are monofunctional reactants capable of reaction with only one amino group of the protein to form a silanol-terminated chain; two or more of such chains can form siloxane cross-links by condensation of silanol groups attached to different chains.

It is not necessary for all of the silicone component to form cross-links with the protein component. The copolymer according to the invention may contain silicone-containing chains attached to the protein components at either end or at both ends, the proportions of single chains to cross-links being controlled by the choice and amount of the reactants and by the reaction conditions. Additional crosslinking may occur on concentrating or drying solutions of the copolymer.

The organofunctional silicone/silane reactant is preferably soluble in a solvent common to the protein for efficient reaction. The conditions for reaction of the organofunctional silicones/silanes with the protein to achieve the desired degree of reaction and crosslinking must be carefully controlled, but will be apparent to or readily determinable by those skilled in the art of protein modification.

For reaction of the protein amino groups to occur, it is usually necessary for the pH of the system to be above pH 7 and preferably within the pH range 8 to 11.5. The reaction may be carried out at room temperature, but an elevated temperature and more preferably a temperature of from 40-80°C is preferably used.

The protein component of the protein-silicone copolymers may represent from 5% to 98% of the copolymer, but more preferably 50 to 90% by weight. The protein-silicone copolymers may be supplied as solutions in a suitable solvent, as dispersions or emulsions or as powder.

The chemical structure of the protein-silicone copolymers according to the invention are complicated and it is not possible to write a single general structural formula for these compounds. The silicone component of the protein-silicone copolymers may be illustrated by simplified structures V and VI below, depending upon whether the silicone constitutes a crosslink (V) or a chain attached to the protein at one end (VI). 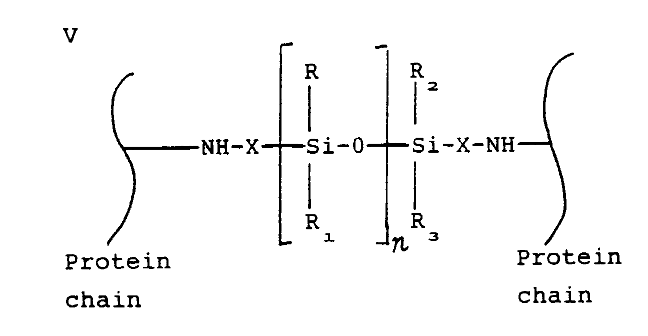 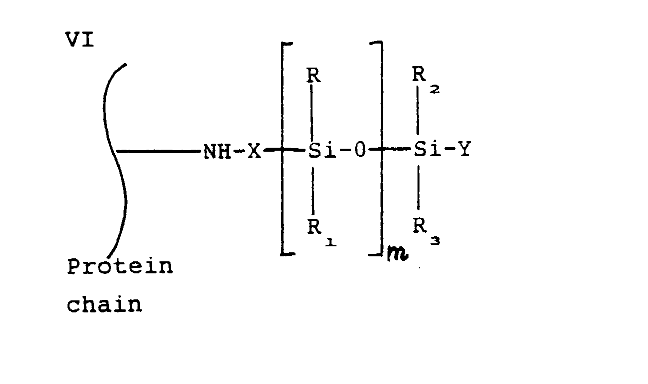 where:-
(a) the protein chain represents a protein or hydrolysed protein which may be chemically modified. Such chemical modification may be performed prior or subsequent to the production of the protein-silicone copolymer. The average molecular weight of the protein is preferably from 500 to 500,000 Daltons;
(b) X is the linking group resulting from reaction of protein amino groups and the amino-reactive group on the organofunctional silicone/silane reactant.
   X may, for example, be: 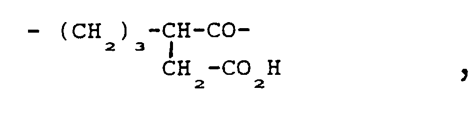 or 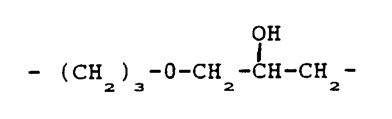
(c) R, R₁, R₂, R₃ may be - CH₃ or -OH,
(d) Y = OH or -X-OH,
(e) n = 1-50; m = 0-50

It will be apparent that the protein-silicone copolymers may contain several silicone chains per protein molecule, depending upon the molecular weight of protein, the proportion of silicone to protein and the degree of cross-linking. The copolymers can be characterised in terms of both chemical and physical properties by standard techniques and the degree of modification of protein amino groups and the protein and silicone contents determined.

The protein-silicone copolymers according to the invention and described above have been found to exhibit physical and chemical properties which make them valuable for cosmetic formulations used for conditioning of hair or skin. The properties vary with the composition of the protein-silicone copolymer, but specific benefits which have been found include: solubility in aqueous, aqueous/alcoholic or alcoholic solutions, self-cross-linking (polymerisation) behaviour on drying to a film, enhanced substantivity to skin and hair, moisture retention, novel feel on skin, and excellent wet and drying combing behaviour on hair.

The inclusion of the protein-silicone copolymers into cosmetic formulations can be at any desired level; it is generally preferred to use from 0.1 to 5% w/w of the copolymer.

In the copolymers of the invention, carboxyl groups of the protein component and/or amino groups of the protein component which are not bonded to the silicone component, can be chemically modified by reaction with a non-silicone, eg. by esterification, acylation or quaternisation.

In order that the invention may be more fully understood, the following examples, in which all percentages are by weight, are given by way of illustration only:

### Example 1

A protein-silicone copolymer was prepared by reaction of an organofunctional silane with hydrolysed collagen protein having a weight average molecular weight of approximately 5,000 Daltons. The product had the general structure illustrated in V and VI where:
R = CH₃
R₁ = OH
R₂ = CH₃
R₃ = OH
X = 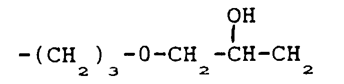
Y = OH
n = 1,m = 0

The degree of modification of the protein amino groups was 48% and the protein content of the copolymer was 90%. The product was significantly more viscous than the original collagen hydrolysate at equivalent concentration, as a consequence of partial cross-linking as indicated by the figures below:-

| | Viscosity at 25%, 25°C |
|---|---|
| Hydrolysed Collagen | 30 mps |
| Collagen-Silicone Copolymer | 500 mps |

The copolymer was soluble in water and 50/50 alcohol-water.

Silicon NMR (nuclear magnetic resonance) studies on the copolymer identified the presence of peaks attributable to both silanol and siloxane groups; that is, single chains and cross-linking chains.

When applied as a solution to a glass-slide and dried, the copolymer produced a hard, glossy (non-tacky) film. Attempts to redissolve the film in water at 25°C resulted in only 90% dissolution of the copolymer. In contrast, the parent hydrolysed collagen treated similarly dissolved completely and much more rapidly. This behaviour of the copolymer was attributed to the formation of cross-links.

When the copolymer was applied to the skin as a 5% solution it exhibited the characteristic tack of hydrolysed collagen, but with a pleasing lubricating feel and when dry conferred a smooth feel to the skin.

When a hair swatch was treated by immersing it in a 2% solution of the copolymer for 30 seconds followed by rinsing with water, the hair exhibited excellent wet-combing behaviour when compared with a water-control. Radiolabelling studies of substantivity to hair showed that the copolymer exhibited behaviour characteristic of proteins, but with enhanced substantivity compared with the parent hydrolysed collagen.

### Example 2

A protein-silicone copolymer was prepared by reaction of an organofunctional silane with hydrolysed wheat protein having a weight average molecular weight of approximately 10,000 Daltons. The product had the general structure illustrated in V and VI where:-
R = OH
R₁ = OH
R₂ = OH R₃ = OH
X = 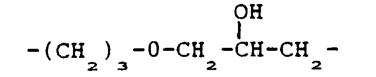
Y = OH
n = 1, m = 0

The degree of modification of the protein amino groups was 60%. The remaining amino groups were then quaternised to attach lauryl dimethyl quat groups to the copolymer, bringing the total modification of amino groups of 85%.

The resultant protein-silicone copolymer (quaternised) exhibited inverse solubility in water. That is, it was more soluble at high active concentrations in water than at low active concentrations. The effect was very pH dependent. At PH 7, a haze developed at concentrations below approximately 10% concentration; at pH 4.5 the haze point was at approximately 20% concentration at pH 9 the haze point was at approximately 1% concentration.

The viscosity of the copolymer was significantly increased compared with the starting wheat protein at equivalent concentrations, due to crosslinking, as indicated below:

| | Viscosity at 27% w/w, 25°C |
|---|---|
| Wheat Protein | 40 mps |
| Wheat Protein-Silicone Copolymer | 680 mps |

The copolymer was soluble in aqueous solutions of anionic surfactants. For example, a 6% aqueous sodium lauryl ether sulphate solution containing 2% active copolymer at pH 7 was a clear solution.

Substantivity tests performed on hair, using radiolabelled copolymer showed that the copolymer was substantive to hair from this anionic shampoo system. Hair swatches treated in this way exhibited improved wet and dry combability and general manageability.

### Example 3

A protein-silicone copolymer was prepared by reaction of an organofunctional silicone with an ethyl ester of hydrolysed collagen having a weight average molecular weight of approximately 4,000 Daltons. The product had the general structure illustrated in V and VI where:-
R= CH₃
R₁= CH₃
R₂= CH₃
R₃= CH₃
X = 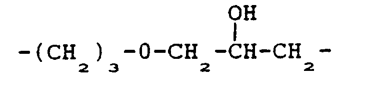
n = 12
The reaction procedure was as follows:-

90g of the ethyl ester of hydrolysed collagen dissolved in 210g absolute ethanol were placed in a 1 litre glass reaction vessel fitted with a stirrer and a reflux condenser. The temperature was increased to 70°C using a water-bath and the pH adjusted to 11.0 with solid sodium hydroxide. 108g of organofunctional silane (reactant of formula III above, n=12) were added and the temperature and pH maintained at 70°C and pH 11.0 respectively overnight. The pH was then reduced by addition of concentrated sulphuric acid to pH 6 and the solution filtered to give a solution of protein-silicone copolymer.

The copolymer contained 45% protein and 70% of the amino groups had been modified.

The alcohol-soluble copolymer is useful for hair sprays, providing a glossy film on the hair. It is insoluble in water and precipitates from alcoholic solution on addition of water.

### Example 4

A protein-silicone copolymer was prepared by reaction of an organofunctional silane with hydrolysed wheat protein having a weight average molecular weight of approximately 3,500 Daltons. The product had the general structure illustrated in V and VI, where:
R= OH
R₁= CH₃
R₂= OH
R₃= CH₃
X = 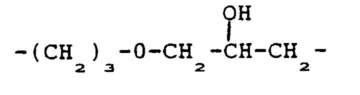
Y = OH
n = 1, m = 0

The degree of modification of the protein amino groups was 82% and the protein content of the copolymer was 50%.

The viscosity of the copolymer, measured at 27% w/w concentration, 25°C was 400 mps, which represented a substantial increase over the viscosity of the parent protein hydrolysate, due to cross-linking.

The copolymer exhibited inverse solubility in water in the pH region below approximately pH6.

On drying a solution of the copolymer having a pH of 7 to form a constant weight film at 25°C, 40% RH, additional cross-linking occurred so that the copolymer could only be redissolved to the extent of 67% in pH7 buffer at 25°C. A similar film formed from a pH4 solution could only be redissolved to the extent of 62%. In contrast, control films of the parent wheat protein hydrolysate dissolved completely and much more rapidly.

Hair swatches were treated with 3% active solutions of the copolymer at pH7 and then rinsed with water. The wet combability was assessed by eight panellists, in comparison with tresses similarly treated with the parent hydrolysed wheat protein. The hair tresses were subsequently dried and assessed for dry combability. The tresses treated with the copolymer were found to be significantly better on both wet and dry combability than the control tresses treated with the wheat protein hydrolysate.

1% of the copolymer was added to a conventional shampoo formulation which was used to treat hair tresses. The contact time was 10 minutes, after which the tresses were thoroughly rinsed with water and dried. Control tresses were treated with the same shampoo containing no copolymer.

24 panellists assessed the hair tresses for feel, shine and dry-combing behaviour. Tresses treated with shampoo containing copolymer were scored higher than the control tresses in terms of feel, shine and dry-combability.

## Claims

1. A protein-silicone copolymer wherein the silicone component is covalently bonded to amino groups of the protein and at least some of the silicone component forms cross-links between different protein chains, the protein component forming from 5% to 98% by weight of the copolymer.

2. A copolymer according to claim 1, wherein the protein component forms from 50% to 90% by weight of the copolymer.

3. A copolymer according to claim 1 or 2, wherein the protein component has a weight average molecular weight of from 500 to 500,000 Daltons.

4. A copolymer according to any of claims 1 to 3, wherein carboxyl groups of the protein component and/or amino groups of the protein component which are not bonded to the silicone component, have been chemically modified by reaction with a non-silicone.

5. A copolymer according to claim 4, wherein said modification has been effected by esterification, acylation or quaternisation.

6. A copolymer according to any of claims 1 to 5, wherein the protein component is collagen, elastin, keratin, casein, wheat protein, soya protein, or silk.

7. A copolymer according to any of claims 1 to 6, which comprises cross-links of the structure: 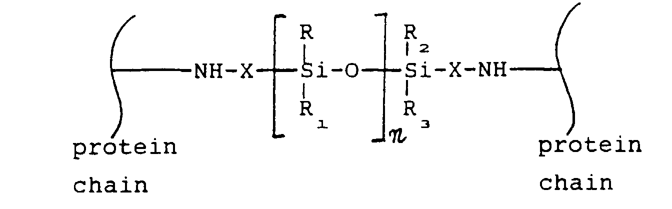 where X is a linking group of the formula: 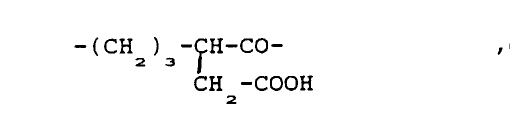 or
-(CH₂)₃-O-CH₂-CH(OH)-CH₂-,
R, R₁, R₂ and R₃, which may be the same or different, are -CH₃ or -OH, and
n is an integer from 1 to 50.

8. A copolymer according to claim 7, which additionally comprises free siloxane groups of the structure: 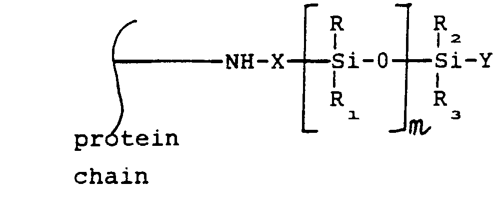 where X, R, R₁, R₂ and R₃ have the meanings stated in claim 6; Y is -OH or -X-OH; and m is an integer up to 50.

9. A cosmetic formulation which comprises a copolymer as claimed in any of claims 1 to 8.

10. A cosmetic formulation according to claim 9, which comprises from 0.1 to 5% w/w of the copolymer.

## Patentansprüche

1. Ein Protein-Silicon-Copolymer, wobei die Siliconkomponente durch kovalente Bindungen an Aminogruppen des Proteins gebunden ist und mindestens ein Teil der Siliconkomponente zwischen verschiedenen Proteinketten Vernetzungen bildet, wobei die Proteinkomponente 5% - 98 Gew.-% des Copolymers bildet.

2. Ein Copolymer nach Anspruch 1, wobei die Proteinkomponente 50 bis 90 Gew.-% des Copolymers bildet.

3. Ein Copolymer nach Anspruch 1 oder 2, wobei die Proteinkomponente ein Durchschnittsmolekulargewichts (Gewichtsmittel) von 500 bis 500.000 Dalton aufweist.

4. Ein Copolymer nach einem der Ansprüche 1 bis 3, wobei Carboxylgruppen der Proteinkomponente und/oder Aminogruppen der Proteinkomponente, welche nicht an die Siliconkomponente gebunden sind, durch Reaktion mit einem Nichtsilikon chemisch modifiziert worden sind.

5. Ein Copolymer nach Anspruch 4, wobei die Modifikation durch Esterifizierung, Acylierung oder Quaternisierung bewirkt worden ist.

6. Ein Copolymer nach einem der Ansprüche 1 bis 5, wobei es sich bei der Proteinkomponente um Collagen, Elastin, Keratin, Casein, Weizenprotein, Sojaprotein oder Seide handelt.

7. Ein Copolymer nach einem der Ansprüche 1 bis 6, welche Quervernetzungen folgender Struktur enthält: 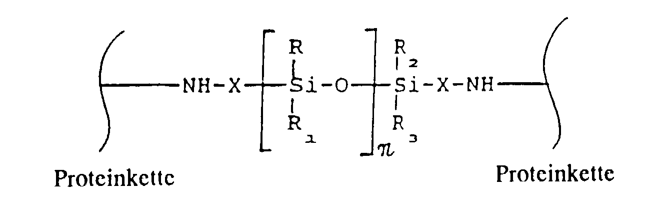 wobei X eine Vernetzungsgruppe folgender Formel darstellt: 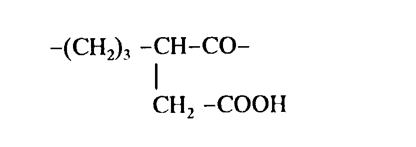 oder
-(CH₂)₃-O-CH₂ -CH(OH)-CH₂-
R, R₁, R₂, R₂ und R₃, welche gleich oder verschieden sein können, -CH₃ oder -OH bedeuten und
n eine ganze Zahl von 1 bis 50 darstellt.

8. Ein Copolymcr nach Anspruch 7, welches zusätzlich freie Siloxangruppen folgender Struktur umfaßt: 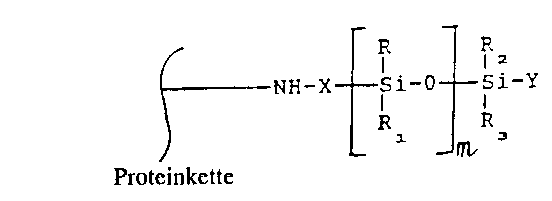 wobei X,R,R₁, R₂ und R₃ die in Anspruch 6 angegebene Bedeutung besitzen; Y für - OH oder -X-OH steht und m eine ganze Zahl von bis zu 50 bedeutet.

9. Eine kosmetische Rezeptur, welche ein Copolymer nach einem der Ansprüche 1 bis 8 enthält.

10. Eine kosmetische Rezeptur nach Anspruch 9, welche 0,1 bis 5 Gew.-% des Copolymers enthält.

## Revendications

1. Un copolymère de protéine et de silicone où la composante silicone est liée de manière covalente aux groupes amine de la protéine et où au moins une partie de la composante silicone forme des réticulations entre les différentes chaînes de protéines, la composante protéine représentant 5 à 98 % en masse du copolymère.

2. Un copolymère conforme à la revendication 1, où la composante protéine constitue entre 50 et 90 % en masse du copolymère.

3. Un copolymère conforme à la revendication 1 ou 2, où la composante protéine a une masse moléculaire moyenne de 500 à 500.000 Daltons.

4. Un copolymère conforme aux revendications 1 à 3, où les groupes carboxyles de la composante protéine et/ou ses groupes amine non liés à la composante silicone, ont été modifiés chimiquement par réaction avec un non-silicone.

5. Un copolymère conforme à la revendication 4, où ladite modification a été réalisée par estérification, acylation ou quaternisation.

6. Un copolymère conforme aux revendications 1 à 5, où la composante protéine est le collagène, l'élastine, la kératine, la caséine, la protéine de froment, la protéine de soja ou la soie.

7. Un copolymère conforme aux revendication 1 à 6, comportant des réticulations de la structure : 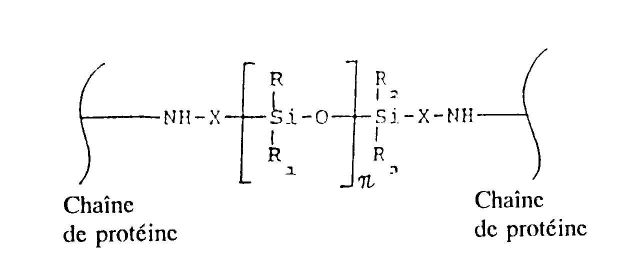 où X est un groupe de liaison suivant la formule : 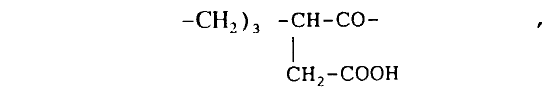 ou
-(CH₂)₃-O-CH₂-CH(OH)-CH₂-
R, R₁, R₂ et R₃, qui peuvent être identiques ou différents, sont -CH₃ ou -OH et
n est un entier de 1 à 50.

8. Un copolymère conforme à la revendication 7, qui comporte en outre des groupes siloxane libres de cette structure : 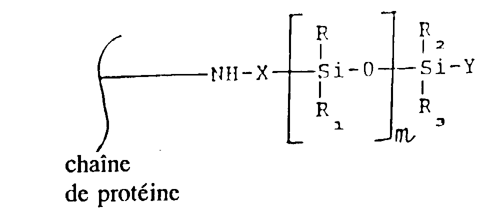 où X, R, R₁, R₂ et R₃ correspondent à ce qui est indiqué à la revendication 6 ; Y étant -OH ou -X-OH et m un entier dont le maximum est 50.

9. Une formulation cosmétique contenant un copolymère tel que revendiqué dans les revendications 1 à 8.

10. Une formulation cosmétique conforme à la revendication 9 contenant de 0,1 à 5 % en masse du copolymère.
